# EUROPEAN PATENT SPECIFICATION

(11) **EP 0 680 333 B1**
(45) Date of publication and mention of the grant of the patent: **06.05.1999**
(21) Application number: 94906142.8
(22) Date of filing: 21.01.1994
(51) Int. Cl.: A61K 38/46, A61K 39/395, A61K 38/55, A61K 31/40, A61K 31/44, A61K 31/47, A61M 1/38, G01N 33/569, G01N 33/573, C12N 15/57

(54) **PREVENTION AND TREATMENT OF CYTOMEGALOVIRUS USING AMINOPEPTIDASE**
VERHÜTUNG UND BEHANDLUNG VON ZYTOMEGALOVIRUS MITTELS AMINOPEPTIDASE
PREVENTION ET TRAITEMENT DE CYTOMEGALOVIRUS PAR L'AMINOPEPTIDASE

(30) Priority: 21.01.1993 US 6982; 30.07.1993 US 99664
(43) Date of publication of application: 08.11.1995
(73) Proprietor: MÖLLER, Erna, S-133 33 Saltsjobaden (SE); SÖDERBERG, Cecilia, S-126 50 Hargersten (SE)
(72) Inventor: ZAIA, John A., Arcadia, CA 91007 (CA); GIUGNI, Terrence, Alta Loma, CA 91701 (CA); MÖllER, Erna, S-133 33 Saltsjobaden (SE); SÖDERBERG, Cecilia, S-126 50 Hargersten (SE)
(74) Representative: Dzieglewska, Hanna Eva
(86) International application number: PCT/EP94/00188
(87) International publication number: WO 94/16724

(56) References cited:
- NATURE. vol. 357 , 4 June 1992 , LONDON GB pages 420 - 422 C.L. YEAGER ET AL. 'HUMAN AMINOPEPTIDASE N IS A RECEPTOR FOR HUMAN CORONAVIRUS 229E.' cited in the application
- NATURE. vol. 357 , 4 June 1992 , LONDON GB pages 417 - 420 B. DELMAS ET AL. 'AMINOPEPTIDASE N IS A MAJOR RECEPTOR FOR THE ENTERO-PATHOGENIC CORONAVIRUS TGEV.' cited in the application
- JOURNAL OF VIROLOGY vol. 67, no. 6 , June 1993 , BALTIMORE, MD, US pages 3166 - 3175 C. SÖDERBERG ET AL. 'DEFINITION OF A SUBSET OF HUMAN PERIPHERAL BLOOD MONONUCLEAR CELLS THAT ARE PERMISSIVE TO HUMAN CYTOMEGALOVIRUS INFECTION.' cited in the application
- JOURNAL OF VIROLOGY vol. 67, no. 11 , November 1993 , BALTIMORE, MD, US pages 6576 - 6585 C. SÖDERBERG ET AL. 'CD 13 (HUMAN AMINOPEPTIDASE N) MEDIATES HUMAN CYTOMEGALOVIRUS INFECTION.'
- JOURNAL OF GENERAL VIROLOGY vol. 71, no. 4 , April 1990 , COLCHESTER, GB pages 751 - 766 T.L. LENTZ 'THE RECOGNITION EVENT BETWEEN VIRUS AND HOST CELL RECEPTOR: A TARGET FOR ANTIVIRAL AGENTS.'

## Description

This invention relates to the use of aminopeptidases (AP), preferably human aminopeptidase N (APN), and antibodies against these, for the diagnosis prevention and treatment of human cytomegalovirus (CMV) infections.

Human cytomegalovirus is responsible for significant morbidity and mortality in immunocompromised groups, i.e., neonates, organ transplant recipients, AIDS patients (1-3). Treatment is limited because the early events of CMV infection, involving cell binding and penetration, are largely unknown (4). At least four different cell surface proteins, a 30-34 K protein(s) (5-7), a 92.5 K protein (8), a heparin-binding protein (9), and class I human leukocyte antigen (10), have been suggested as important in virus binding.

It has recently been reported that CD13 (human aminopeptidase N, APN) is expressed on blood cells susceptible in vitro to HCMV infection (11,12,13).

APN is a metalloprotease present on apical surfaces of epithelial cells (14-17). It has been reported to be a binding protein for certain coronaviruses (18,19).

CD4 is a T-cell receptor for the HIV virion surface glycoprotein gp 120 which also migrates to the surface of HIV infected cells. Soluble forms of CD4 have been developed for circulation in the blood to bind both HIV and infected T-cells and thus prevent the virus from infecting new T-cells. See, e.g., Research News, 1559-1560 (1989).

We have discovered that AP, including aminopeptidase N and fragments thereof, are important in CMV infection. It is surprisingly present both on CMV virions and on the cell surface and mediates critical event(s) of infection. It is thus possible to combat infection of cells by CMV by blocking the AP site on either the cells or the virions or both, for example by administration of AP or a fragment thereof, an antibody against AP or an effective fragment thereof or an AP inhibitor.

The method for the prevention or treatment of human cytomegalovirus (HCMV) infection comprises administering to a patient in need thereof an amount of an aminopeptidase, a fragment of an aminopeptidase, an inhibitor of an aminopeptidase and/or an antibody against an aminopeptidase therapeutically effective to provide such prevention or treatment.

The aminopeptidase is preferably human aminopeptidase N, particularly in cell free form.

An aspect of the invention includes neutralization or mediation of CMV infection by the use of an AP antibody to remove CMV from body fluids such as blood products and from bone marrow by binding to the virions. Bone marrow transplant (BMT) recipients may receive marrow or blood products which have been purged by treatment with either free or immobilised antibody to AP or free or immobilised water soluble AP (sAP) linked to a solid support to remove both CMV virions and cells which are susceptible to and thus may harbor and transmit CMV infection. Such cells form a relatively small sub-population and may eventually be replaced by differentiation of healthy stem cells.

An important aspect of the invention is the discovery that sAP inhibits or neutralizes CMV infection. sAP thus functions as an antiviral agent. Pursuant to this aspect of the invention, sAP or truncated forms thereof which contain the domain which mediates infection after CMV is bound to the cell are administered exogenously to a patient or expressed as a polypeptide by the cells of a CMV patient. In like manner, AP or truncated forms thereof may be administered prophylactically prior to infection. For example, AP preemptive therapy may be provided for management of patient populations such as AIDS patients, bone marrow transplant or organ transplant recipients at risk for CMV disease. The procedure is similar to that previously used for ganciclovir (20).

The invention also refers to the administration of antibodies to aminopeptidases and the administration of enzyme inhibitors of aminopeptidase as well as combinations of an aminopeptidase and an antibody thereto or an enzyme inhibitor thereof.

The invention is described by way of illustration only with reference to the following drawings in which:

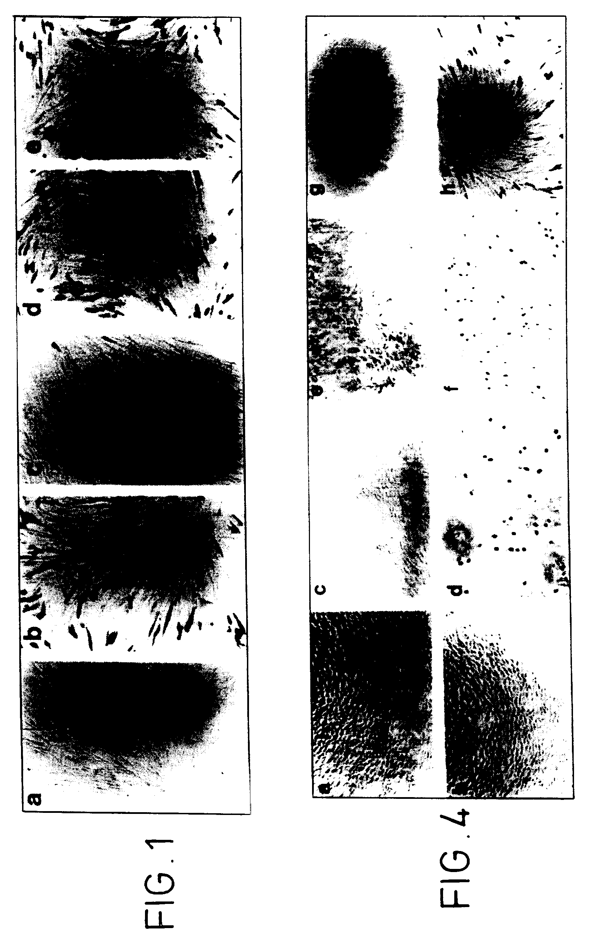

Figure 1. Inhibition of CMV Towne-strain (RC256) infection of HL734 cells by U71, an APN specific monoclonal antibody. HL734 cells were uninfected (a) or infected with CMV RC256 strain encoding the bacterial β-galactosidase gene in the absence (b) or presence of an APN specific antibody (c), control mouse ascites (d) or a polyclonal antibody to the epidermal growth factor-receptor (e).
   Methods. Human embryonic lung fibroblasts (HL734) grown in 96-well plates were infected with CMV RC256 in the absence or presence of various antibodies. Cells were incubated with RC256 at MOI = 1 for 2 hours at 4°C, washed, and incubated at 37°C for 16 hours. The cells were fixed in 1% phosphate-buffered glutaraldehyde for 15 minutes, washed, and assayed for the expression of β-galactosidase by incubation with Bluo-Gal (430 µg/ml) (Gibco/BRL) for 16 hours at 37°C. An APN-specific antibody (U71), control mouse ascites or a rabbit anti-human epidermal growth factor-receptor serum were added during incubation of cells with virus. Infected cells were detected by the presence of precipitated blue substrate in cytoplasm of cells as visualized by light microscopy.

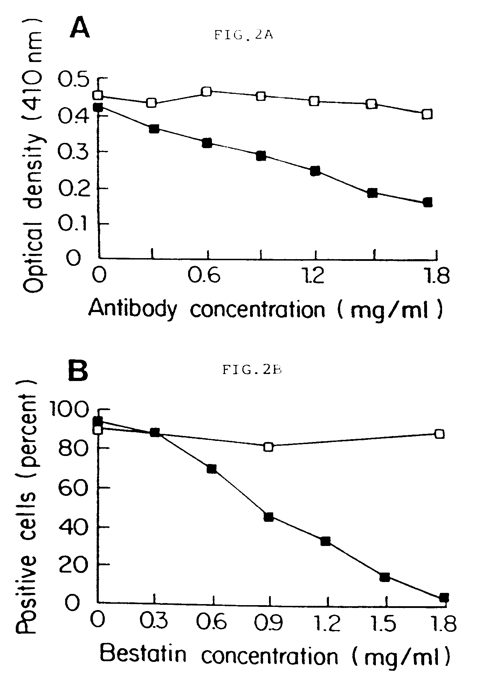

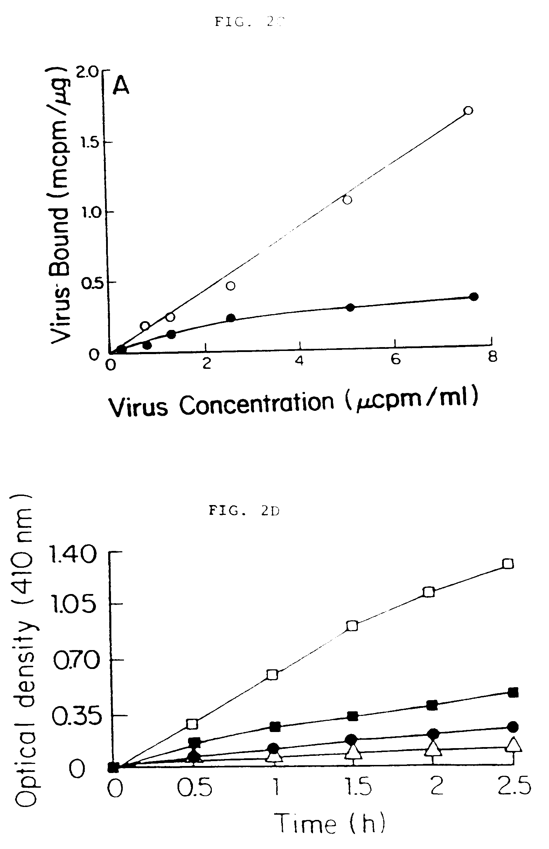

Figure 2. Ability to inhibit infection by compounds that interact with human aminopeptidase N. Panel a: HL734 cells were infected with CMV strain RC256 in the presence of serial dilutions of either U71 (■) or control mouse ascites (□). Infection was quantitatively determined by spectrophotometric measurement of the cleavage of p-nitrophenyl-β-D-galactopyranoside by β-gal. Panel b: HL734 were infected with CMV strain AD169 (■) or Semliki Forest Virus (SFV) (□) in the presence of increasing concentration of bestatin. Infection was quantitatively determined by immunocytochemistry using an antibody to the mIE protein of CMV or immune serum to SFV and enumeration of infected cells. Panel c: the binding of ³⁵S-HCMV to MRC-5 cells in the presence of mouse IgG (o) or antibody to APN (●). Panel d: the aminopeptidase N activity on the surface of HL734 (■), NIH-3T3 (●), hAPN-3T3 (□), or hAPNmut-3T3 (△) cells expressed as optical density of reaction product p-nitroanilide.
   Method. Infection of HL734 with CMV RC256 was performed as described in Figure 1 in the presence of serial dilutions of either U71 or a control mouse ascites. Cells were washed, fixed with glutaraldhyde and assayed for β-gal expression by incubation with p-nitrophenyl-β-D-galactopyranoside (1 mg/ml) and the production of p-nitrophenol was measured spectrophotometrically at 410 nm. To measure the effect of aminopeptidase inhibitors on viral infection, HL734 were preincubated with increasing concentrations of bestatin for 1 hour at 37°C, infected with CMV strain AD169 at an MOI = 1 or SFV at an MOI = 30 for 1 hour at 37°C, washed, and incubated in fresh media for six hours at 37°C. After fixation in ice cold acetone/ methanol (1/1), CMV nuclear antigens were detected with a mouse monoclonal to the 72K CMV mIE protein (NEW/ Dupont, Inc.) followed by a phycoerythrine-labelled F(ab')₂ fragment of rabbit-anti mouse IgG (Dakopatts, Inc). Cells infected with SFV were demonstrated using a rabbit polyclonal immune serum against SFV followed by a rhodamine-conjugated goat anti-rabbit IgG (Biosystems, Inc.). Binding of HCMV to MRC-5 cells was carried out by incubating increasing concentration of ³⁵S-HCMV with MRC-5 cells for 2 hours at 4°C. Incubation was carried out in the presence of mouse IgG (Sigma, Inc.) or L138 (Becton Dickinson, Inc.) at a concentration of 16 µg/ml. Cells were washed five times with hepes-buffered saline, solubilized with 0.5 M NaOH, and cell-associated ³⁵S-labelled virions was measured quantitatively by liquid scintillation counting. Aminopeptidase activity on intact cells was measured by plating 1-5 x 10⁴ cells in 96-well plates, incubating overnight in fresh media, and then incubating with alanine-p-nitroanilide (6 mM) at 37°C. At various times, the free p-nitroanilide released was measured spectrophotometrically at 410 nm. The aminopeptidase activity was normalized to cell number measured by BCA protein assay (Pierce). All measurements were determined in triplicate.

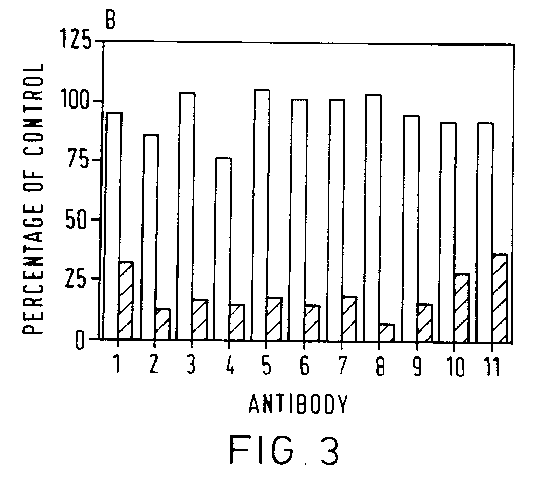

Figure 3. Inhibition of CMV infection of HL734 cells with antibodies to human CD13 (HAPN).

Monoloayer culture of HL734 cells were infected with HCMV AD169 in the presence of one of eleven different APN specific antibodies at 4°C for two hours and the cells were washed and incubated in complete medium at 37°C overnight. Detection of infected cells was by assaying for the expression of HCMV mIE as described in relation to Figure 1. The results are shown as closed bars. The experiment was repeated with pre-incubation of the cells with the antibodies at the concentrations stated below for 1 hour at 4°C and removal of excess antibody by washing. The results are shown in Figure 3 as open bars.

The following APN specific monoclonal antibodies were used at the concentrations stated:
(1) WM15; 105 µg IgG/ml (obtained from E. Favoloro, Westmead Hosp. N.S.W., Australia)
(2) MY7; 100 µg IgG/ml (obtained from J.D. Griffin, Dana Faber Cancer Inst., Boston, USA)
(3) 43E6; 220 µg IgG/ml (both obtained from H.J. Buhring, Medizische Klinik
(4) 46A11; 320 µg IgG/ml II, Tübringen, Germany)
(5) 72a; 770 µg IgG/ml (obtained from R.F. Todd University of Michigan Med. Centre Ann. Arbor, Mich.)
(6) MCS-2; 1 mg IgG/ml (obtained from K. Sagawa, Kurume University, Japan)
(7) 22A5; 13.2 mg protein/ml (obtained from M.A. Horton, St. Bartholomews Hosp., London, UK)
(8) 3D8; 3 mg protein/ml (obtained from A.E. Koch, Northwestern Univ. Med. School, Chicago)
(9) RMAG-6; 1.9 mg IgG/ml (obtained from P.J. O'Connell, Royal Melbourne Hosp. Victoria, Australia)
(10) L138; 22 µg IgG/ml (= Anti-LeuM7 obtained from Becton Dickinson, San Jose, California)
(11) WM47; 90 µg IgG/ml (obtained from Dakopatts, Glostrup, Denmark)

It will be seen that preincubation of the cells with the APN specific antibody followed by washing resulted in very little effect on the inhibition of HCMV infection; MY7 and 46A11 had an inhibiting effect of, respectively, 15% and 20% as compared to a 60-90% decrease when any of the APN specific antibodies were was included with the virus inoculum during infection.

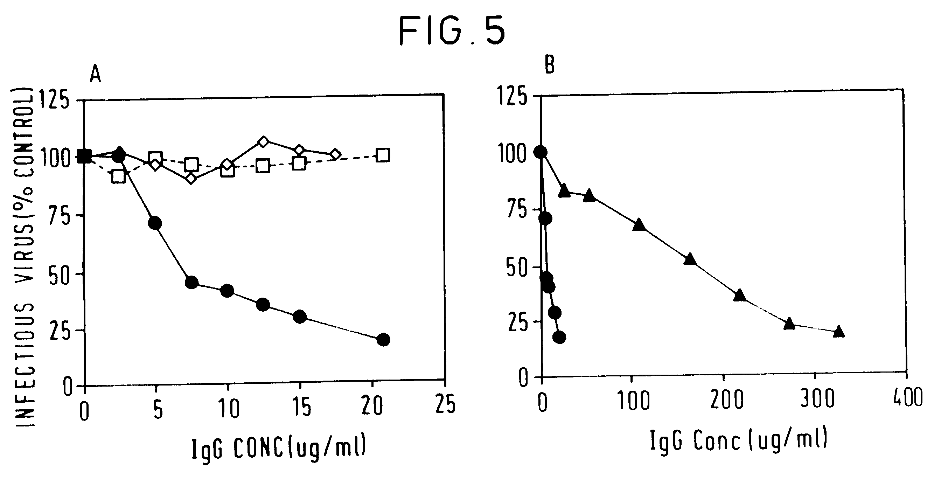

Figure 4. Susceptibility to in vitro CMV infection of human fibroblasts and mouse cells expressing human APN. Parental murine NIH-3T3 cells (panels a and b), transfected NIH-3T3 cells expressing high levels of native human APN (hAPN-3T3) (panels c and d) or mutant human APN (hAPNMUT-3T3; 15,21) which lacks 39 amino acids at positions 360-399 including the zinc-binding site at positions 388-392 necessary for enzyme activity (panels e and f), and human HL734 (panels g and h) were uninfected (panels a, c, e and g) or infected (panels b, d, f and h) with CMV RC256. Infection was performed and assayed as described in the legend to Figure 1.

Figure 5. Neutralisation of HCMV AD169. HCMV AD169 was preincubated with antibodies with specificity to HAPN, L138 (●), to a human fibroblast surface antigen IB10 (◇), to mouse IgG (□), or to HCMV gB (7-5) ( 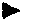 , as a positive control) immobilised on protein A sepharose CL-4B or with protein A sepharose alone as a control (40 µl of a 50% slurry added to a final volume of 300 µl, Pharmacia/LKB, Uppsala, Sweden) in binding buffer. (20mM Tris, pH 7.4, 150 mM NaCl, 1 mg/ml BSA). After 1 hour of incubation at 4°C with continuous agitation, the protein A sepharose/antibody complexes were pelleted by centrifugation for 2 minutes in an Eppendorf microcentrifuge. The supernatant was used to inoculate monolayers of HL734 cells by incubation for 2 hours at 4°C, washed and incubated at 37°C overnight with complete medium. The quantity of virus remaining in the supernatant following precipitation with antibody was assayed by determining the number of cells expressing the mIE protein and normalising its value to the values obtained for the protein A sepharose control.

The HAPN-specific antibody (L138) eliminated HCMV in dose-dependent fashion whereas equal amounts of mouse IgG or the antibody specific to HFF surface antigen (1B10) had no effect on the infectibility of the HCMV as is shown in Panel A. As shown in Panel B, the anti-HAPN antibody eliminated HCMV from the solution with an ED₅₀ of 7.1 µg/ml compared to 760 µg/ml for the HCMV gB specific antibody.

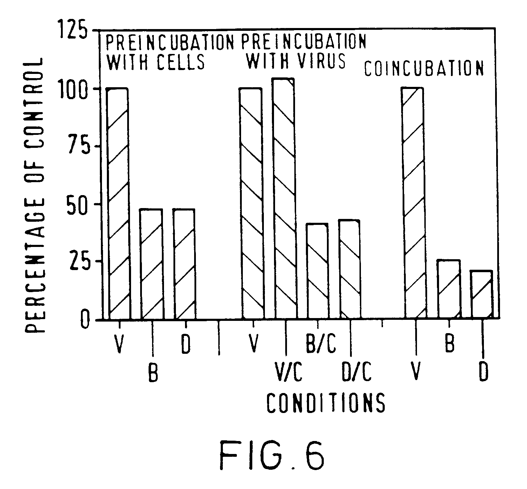

Figure 6. Inhibition of HCMV infection by preincubation of virus or cells with aminopeptidase inhibitors.

Preincubation with cells: HL734 cells were incubated with bestatin (3.5 mM) or 2,2'-dipyridyl (5.4 mM) for 1 hour at 4°C, washed, incubated with HCMV AD169 for 2 hours at 4°C, washed, incubated for 16 to 18 hours at 37°C in complete media and assayed for HCMV infection as described in the legend to Figure 1. The assay results are shown as V = untreated cells infected with untreated virus, B = preincubation with bestatin, D = preincubation with 2,2'-dipyridyl.

Preincubation with virus: An inoculum of HCMV was incubated with bestatin or 2,2'-dipyridyl for 1 hour at 4°C and separated from free drug by fractionation on a PD=10 column, and the eluate was used to inoculate HL734 cells as in the legend to Figure 1. Infection of HL734 cells and assay for HCMV infection were as described above. The results are shown as V/C = virus preincubated without drug and fractionated over PD10 column (Bio Rad, Richmond, California), B/C = virus preincubated with bestatin, D/C = virus preincubated with 2,2'-dipyridyl.

Coincubation: HL734 cells also were infected with HCMV in the presence of bestatin or 2,2'-dipyridyl (coincubated) as described in the legend to Figure 1. The results are shown as V = untreated cells infected with untreated virus, B = incubation in presence of bestatin, D = incubation in presence of 2,2'-dipyridyl.

Bestatin and 2,2'-dipyridyl inhibited infection by 50% when incubated with cells prior to infection (Fig. 5, preincubation with cells, column B and D) at a concentration that gave 75 to 80% inhibition when incubated with the cells during inoculation with the virus (Fig. 5, coincubation, column B and D). The incubation of the virus inoculum with the inhibitors also resulted in a 50% decrease in infectivity of the virus (Fig. 5, preincubation with virus, column B/C and D/C). The fractionation of HCMV over a PD-10 column had no effect on its infectability (Fig. 5, preincubation with virus, compare column V with column V/C). It can thus be seen that AP inhibitors block HCMV infection by interacting with both the virus and the host cells and that the effect of the inhibitors is additive if they are incubated with both the virus and the host cell.

This invention thus relates to a number of aspects of the involvement of aminopeptidases in human CMV infection. Antibodies to AP block binding of CMV virions to susceptible cells by binding to and thus neutralising CMV virions. CMV-resistant murine fibroblasts become susceptible to CMV infection after transfection with complementary DNA encoding human APN. However, murine fibroblasts transfected with mutant APN, lacking the enzymatic domain, are also susceptible to CMV infection. Thus, cell surface APN appears to mediate CMV infection but the enzymatic domain of the cell surface expressed APN is not necessary for infection. Surprisingly, however, compounds which inhibit aminopeptidase activity completely block CMV infection.

To investigate the role that APN plays in the early interaction of CMV with human fibroblasts, cell strains HL734 and MRC-5 were exposed to virus in the presence of APN-specific monoclonal antibodies U71 and U81 (obtained from D. Bourel, Centre Regional de Transfusion, Sanguine, Rennes, France), WM47, or L138.

As shown in Figure 1, U71 protected HL734 from infection with CMV RC256, a recombinant form of CMV Towne strain that encodes the β-galactosidase (β-gal)-gene linked to a CMV early promoter (22). No protection was observed in cells treated with control mouse ascites, mouse IgG or with antibodies directed against the epidermal growth factor-receptor (anti-EGFR) known to be present on the surface of these fibroblasts. The dose response curve of CMV inhibition by U71 indicated that inhibition was directly proportional to the amount of anti-APN antibody used (see Figure 2a).

**TABLE 1**

| **Effect of anti-APN monoclonal antibodies and aminopeptidase inhibitors on CMV infection** | | | |
|---|---|---|---|
| | Inhibition of CMV Infection^{a} | Inhibition of SFV Infection^{b} | Inhibition of aminopeptidase activity^{c} |
| Monoclonal antibodies^{d} | | | |
| U71 | + | ND^{e} | + |
| U81 | + | ND | + |
| WM47 | + | - | - |
| L138 | + | - | - |

| Chemical inhibitors^{d} | | | |
|---|---|---|---|
| actinonin | + (3.3) | - | + |
| bestatin | + (2.5) | - | + |
| 1,10-phenanthroline | + (0.25) | - | + |
| 2,2'-dipyridyl | + (2.8) | - | + |

| | | | |
|---|---|---|---|
| ^{a} The inhibition of CMV infection was determined as described in the description of Figure 2. | | | |
| ^{b} The inhibition of Semliki Forest Virus (SFV) infection was determined as described in the description of Figure 2. | | | |
| ^{c} The inhibition of enzymatic activity was measured using the substrate alanine-p-nitroanilide as described in the description of Figure 2. | | | |
| ^{d} Chemical inhibitors were tested at the concentrations that gave maximum inhibition without toxicity to the cells: actinonin, 5.45 mM; bestatin, 4.35 mM; 1,10-phenathroline, 0.45 mM; 2,2'-dipyridyl, 3.74 mM. The 50% inhibitory dose given in millimolar concentration is listed in parenthesis. Antibodies were tested at a concentration that gave maximum effect: U71, 1.8 mg protein/ml; U81, 2 mg protein/ml; WM47, 20 ug IgG/ml; L138 3.7 ug IgG/ml. + = >50 inhibition compared to mouse IgG control; - = no inhibition. | | | |
| ^{e} ND = not done. | | | |

To investigate whether the enzymatic activity of APN is necessary for CMV infection, four different aminopeptidase inhibitors, actinonin, bestatin, 2,2'-dipyridyl and 1,10-phenanthroline, were evaluated for their effect on enzymatic activity and CMV infection. Bestatin and actinonin are competitive inhibitors of aminopeptidases, and 1,10-phenanthroline and 2,2'-dipyridyl inhibit aminopeptidase activity by chelating zinc required for enzyme function. These compounds were used at concentrations which were demonstrated to inhibit APN in cells using alanine-p-nitronailide as substrate (see Table 1). Toxicity of these substances, measured by trypan blue uptake in cells treated with the inhibitors, was less than 10% in the highest concentrations used (data not shown). As shown in Figure 2b, bestatin inhibited CMV infection at a 50% inhibitory concentration of 2.5 mM. As shown in Table 1, actinonin, 1,10-phenanthroline and 2,2'-dipyridyl inhibited CMV infection with 50% inhibition doses of 3.3, 0.25, and 2.8 mM, respectively, further suggesting that APN is involved in CMV infection.

The effect of APN-specific antibodies on binding of ³⁵S-labeled CMV virions to MRC-5 cells was analyzed. Incubation of cells with increasing concentration of virions resulted in increased binding of CMV to the cells (see Figure 2c). If binding was done in the presence of control mouse IgG, there was no effect on binding (Figure 2c). However, binding in the presence of an APN-specific antibody (L138) resulted in a decrease in CMV binding (see Figure 2c).

To confirm the predominant effect of blocking APN on the virions rather than the cells, 11 anti-APN antibodies were (a) co-incubated with fibroblast cells and CMV and (b) pre-incubated with cells which were then incubated with CMV. The results are shown in Figure 3. In a further experiment it was shown that, while blocking the virion APN is more effective than blocking the cell APN, co-incubation, in which the antibody contacts both the cell APN and the virion APN is the most effective. This, of course, corresponds most closely to the situation in antiviral therapy.

To further investigate the importance of APN and its enzymatic activity during CMV infection, murine cells (NIH-3T3) were transfected with DNA for hAPN (hAPN-3T3) or for a truncated form of the protein lacking 39 amino acids from a region containing the active site (hAPMUT-3T3). The presence of surface hAPN was confirmed on the human cells and on the transfected NIH-3T3 cells by flow cytometric analyses using APN-specific monoclonal antibodies (data not shown). To confirm the level of APN activity in these cells, they were tested for ability to cleave aminopeptidase substrate alanine-p-nitroanilide. Enzymatic activity was present in the hAPN-3T3 and HL734 cells but not in the parent NIH-3T3 or in hAPMUT-3T3 cells (see Figure 2d). All cells were then challenged with CMV RC256, β-gal, the marker for virus infection, was observed in a greater number of the cells which expressed hAPN (see Figure 3). Parental cells showed little or no expression of CMV encoded genes compared to the APN-expressing cells (compare Figure 3b and 3d/3f). Similarly, when evaluated for expression of the 72K major immediate early (mIE) protein of CMV, there was greatly enhanced expression in the transfected cells (see Table 2). Thus, expression of hAPN on murine cells permits infection of the cells by HCMV. Importantly, virus infection was observed in a greater number of cells expressing the truncated form of APN than in those with unmodified hAPN (See Table 2). Infection of these cells expressing the truncated form of hAPN with CMV resulted in a 50-fold increase of CMV-infected cells compared to the hAPN-nonexpressing NIH 3T3 cells which is a 5-fold increase over cells transfected with native hAPN.

**Table 2**

| **HCMV gene expression in murine cell transfectants** | | | | | |
|---|---|---|---|---|---|
| | | HL734 | NIH-3T3 | hAPN-3T3 | hAPNMUT-3T3 |
| mIE | d1 | 19 | <0.25 | 1.7 | 7.6 |
| expression^{a} | d3 | 25 | <0.25 | 2.5 | 10 |
| percent | d7 | 90 | <0.25 | 2.4 | 13 |

| | | | | | |
|---|---|---|---|---|---|
| ^{a} Percent cells expressing the 72-kDa mIE antigen of HCMV were determined 1, 3 or 7 days postinfection with AD169. Monolayers of HL734 cells were inoculated at MOI = 1 for 2 hours at 4°C, washed and incubated in fresh media. At day 1, 3 and 7 postinfection, the cells were fixed and assayed for the expression of HCMV mIE antigen. The expression of mIE nuclear antigen was detected after fixation in methanol/acetone by incubation with a mouse monoclonal antibody directed against the 72-kDa HCMV mIE protein (NEN-DuPont, Boston, MA or Biosoft, Paris, France) followed by a FITC-labelled (F(ab')₂ fragment of rabbit-anti mouse IgG (Dakopatts). Infected cells were detected by using a Nikon microscope fitted with epi-illumination optics. The number of HCMV-antigen positive cells were counted and presented as percent of total cells. | | | | | |

These results suggest that the enzymatic activity of this molecule is not essential for infection and that the truncated form of APN expressed in the hAPN-mut 3T3 cells enhances a cells susceptibility to CMV infection to a greater extent than the native form (compare Figure 3d and 3f and see Table 2).

The mutant form of hAPN, hAPNMUT-3T3, was obtained as follows. The full length human hAPN cDNA subcloned in the pBluescript plasmid was digested with the BstEII restriction endonuclease, which excised an internal 117 base pair (bp) restriction fragment from the cDNA, but did not cut elsewhere in the cDNA or in the vector. The deleted fragment of 39 amino acids (positions 360-399 included the coding sequence for the zinc binding motif (HExxH) at amino acid positions (388-392) known to be necessary for the enzymatic activity (17). DNA, lacking the deleted sequences, was religated correctly and transfected into NIH 3T3 cells by calcium-phosphate precipitation (21). The presence of hAPN on the cell surface was confirmed by immunostaining by monoclonal antibodies and flow cytometric analysis.

The invention thus particularly comprises the use in the method of the invention of an aminopeptidase which lacks the site responsible for aminopeptidase activity while retaining HCMV binding activity. Thus, human aminopeptidase N lacking at least the amino acids at positions 388 to 392, may be used, more particularly human aminopeptidase N lacking the amino acids at positions 360 to 399.

These experiments have shown that CMV infection of fibroblasts, as well as binding of virions to these cells, can be inhibited by different monoclonal antibodies directed against APN as well as by inhibitors of APN activity. However, certain of these APN-specific monoclonal antibodies do not inhibit hAPN activity. Furthermore, enhanced CMV infection occurred in murine cells expressing hAPN and in cells in which the enzymatic site had been deleted as compared to the parental mouse cells. It appears that the enzymatic active site of cell expressed APN is not essential either for anti-APN inhibition of CMV infection or for the infection of APN-expressing murine cells. The APN-inhibiting compounds which blocked infection may act by changing the conformation of the APN molecule, and thus altering a region which is important for infection or by interacting with the virus associated APN (see Figure 6). These data suggest that a non-enzyme domain of cell-expressed APN is important for CMV infection of fibroblasts.

The ability of anti-APN antibodies to bind to APN on the virions permits immobilised antibodies to be used to remove CMV virions from media such as blood products or bone marrow. A demonstration of this is seen in Figure 5.

Soluble AP is preferred for use in the practice of this invention and may be prepared in known manner. For example, soluble aminopeptidase N are obtained by purification from mouse cells expressing human APN. This may be done by a modification of the methodology of Danielsen and Cowell (Danielsen, E.M. and Cowell, G.M. J. Biochem. Biophys. Methods 8: 41-47 (1983)).

Peptides corresponding to various domains of human APN are derived by (1) proteolytic digests of soluble hAPN followed by HPLC purification of the peptides, (2) expression in E. coli, by recombinant DNA methodologies of various regions of hAPN. These E. coli expressed peptides may be purified by HPLC, and (3) synthesis of synthetic peptides.

Table 3 demonstrates that soluble porcine-leucine aminopeptidases block CMV infection in vitro.

**Table 3**

| **Ability of Soluble Aminopeptidases to block CMV Infection**^{**a**} | | |
|---|---|---|
| Concentration | Cytosolic PAP^{b} | Microsomal PAP^{c} |
| 10 ug/ml | 100 | 100% |
| 100 ug/ml | 80 | 80% |
| 586 ug/ml | ND^{d} | 15% |
| 1000 ug/ml | <10% | ND |

| | | |
|---|---|---|
| ^{a} MRC-5 cells, plated in 96 well plates were infected with RC256 in the absence or presence of soluble porcine aminopeptidases (PAP). Cells were incubated with virus plus PAP for two hours at 4°C, washed and incubated 18 hours with fresh media at 37°C. Cells were fixed with 1% glutaraldehyde and assayed for expression of β-galactosidase by incubation with Blue-Gal (430 ug/ml) for 16 hours at 37°C. Infected cells were detected by presence of precipitated blue substrate observed by light microscopy. The number of cells infected under each condition was normalized to cells infected in absence of any aminopeptidase. | | |
| ^{b} Cytosolic PAP = cytosolic porcine leucine aminopeptidase obtained from Sigma Chemical Co., Inc. (Catalogue No. L-9875). | | |
| ^{c} Microsomal PAP = microsomal porcine leucine aminopeptidase obtained from Sigma Chemical Co., Inc. (Catalogue No. L-5006). | | |
| ^{d} ND = not done. | | |

As Table 3 shows, sAPN is an antiviral agent useful for the prevention or treatment of CMV associated diseases.

It has also been determined that preincubation of cells with antibodies to hAPN followed by excessive washing does not block CMV infection, whereas preincubation of the virus with antibodies to hAPN, but not antibodies directed toward other cell surface markers, does block infection. Further, hAPN negative human cells can be transfected with CMV and the infection blocked by interaction of the virions with antibody to hAPN. This suggests that hAPN is not a simple receptor for CMV as suggested by Söderberg, et al. (11-13) and, at least in part, the hAPN on the surface of the virion is, important for CMV infection. However, as indicated by the expression of hAPN in mouse cells and the effect of sAP on CMV infection in fibroblasts, cell associated aminopeptidases play an important role in CMV infection. Indeed, in so-called capping/stripping experiments, APN can be removed from the surface of the hAPN positive cells and the cells do not then become infected on contact with CMV.

The invention also includes diagnosis of CMV infections by immunoassay to determine the presence, in a sample of a body fluid such as blood or plasma, of either anti-CMV antibodies or of CMV virions. It is surprising that anti-CMV antibodies against APN on CMV virions are formed since it might have been expected that the APN would not have been recognised as foreign in view of its expression on human cells. Nevertheless, such antibodies do exist in patients infected with CMV and can provide evidence of CMV infection, although, since other forms of APN may have given rise to the antibodies, for example by an autoimmune response, detection of such antibodies will normally only provide supportive evidence of CMV infection.

The invention thus includes the following methods of use in the diagnosis of CMV infection or effects resulting therefrom:
1) detection or determination of the presence, quantity or quality of anti-APN antibodies by an immunoassay using an aminopeptidase or fragment thereof as a binding partner for said antibodies;
2) detection or determination of the presence or quantity of CMV virions by an immunoassay using soluble aminopeptidase or an antibody against an aminopeptidase as a binding partner for said virions;
3) isolation of cells expressing APN using an antibody against APN as a binding partner for said APN followed by detection or determination of the presence or quantity of CMV virions in the isolated cells.

The presence or quantity of CMV virions may be determined by lysis of the cells and amplyfying the nucleic acids therein by PCR or other techniques using primers specific to virion nucleic acids.

According to a still further aspect, the invention provides a diagnostic kit of use in diagnosis of CMV infection by immunoassay to determine the presence or quantity of anti-APN antibodies or of CMV virions in a sample, comprising free or immobilised aminopeptidase or a fragment thereof as a binding partner for anti-APN antibodies and/or a free or immobilised antibody against aminopeptidase or soluble or immobilised APN as binding partner for aminopeptidase on said virions, said kit including labelling means permitting the presence or quantity of said anti-APN antibodies or CMV virions in said sample to be determined.

Any form of immunoassay can be used, notably competitive binding and sandwich assays. In each case, it will be necessary to label one of the components of the assay to enable a positive result to be detected. In a competitive binding assay, a binding partner for the analyte to be assayed will be immobilised and a known quantity of a labelled form of that analyte will compete with that in the sample for sites on the binding partner, so that the amount of label either immobilised or left free gives an indication of the amount of the analyte in the sample. In a sandwich assay, a binding partner for the analyte will be immobilised and a labelled form of the binding partner or a different binding partner for the analyte will be added to bind to and label the analyte. Thus, in both cases, labelled forms of either AP or anti-AP will be required. However, where anti-AP is to be labelled, it is also possible to use secondary labelling with a labelled secondary antibody which is specific to the anti-AP. Suitable labels include radionucleides, enzymes, fluorescent molecules or colloidal metals. Immobilisation may be by attachment to conventional plates, microtitre wells, dipsticks or particles.

### BIBLIOGRAPHY

1. Zaia, J.A., et al., Hematology/Oncology Clinics N.A. 4: 603-623 (1990).
2. Rubin, R.H., et al., Transplantation 24: 458-464 (1977).
3. Jacobson, M.A., et al., Ann. Intern. Med. 108: 585-594 (1988).
4. Griffiths, P.D., et al., Biochemistry 241: 313-325 (1987).
5. Taylor, H.P., et al., J. Virol 63: 3991-3998 (1990).
6. Adlish, J.D., et al., Virology 176: 337-345 (1990).
7. Nowlin, D.M., et al., J. Virol 65: 3114-3121 (1991).
8. Keay, S., et al., Proc. Natl. Acad. Sci. USA 86: 10100-10103 (1989).
9. Kari, B. et al., J. Virol. 66: 1761-1764 (1992).
10. Grundy, J.E., et al., Gen. Virol. 68: 793-803 (1987).
11. Söderberg, C. et al., Transpl. Proc. 25: 1416-1418 (1993).
12. Söderberg, C. et al., p. 222, 17th International Herpes Virus Workshop, Edinburgh, Scotland, Aug. 1-7, (1992).
13. Söderberg, C. et al., J. Virol. 67: 3166-3175 (1993).
14. Kenny, A.J. et al.; In Kenny, A.J. and Turner, A.J. (eds.) Mammalian Ectoenzymes, Elsevier Scientific Publishing Co., New York, p. 169, (1987).
15. Look, A.T. et al., J. Clin. Invest. 83: 1299-1307, (1989).
16. Noren, O. et al., In P. Desnuelle (ed) Molecular and Cellular Basis of Digestion, Elsevier/North Holland Biomedical Press, Amsterdam, p. 325, (1986).
17. Vallee, B.L. and Auld, D.S. Biochemistry 29: 5647-5659, (1990).
18. Delmas, B. et al., Nature 357: 417-420, (1992).
19. Yeager, C.L. et al., Nature 357: 420-422, (1992).
20. Schmidt, G.M., et al., N. Engl. J. Med. 324: 1057-1059 (1991).
21. Ashmun, R.A. et al., Blood 79: 3344-3349, (1992).
22. Spaete, R.R. et al., Proc. Natl. Acad. Sci. USA 84: 7213-7217, (1987).

## Claims

1. Use of an aminopeptidase, a fragment of an aminopeptidase, an inhibitor of an aminopeptidase and/or an antibody against an aminopeptidase in the manufacture of a medicament for use in the prevention or treatment of human cytomegalovirus (HCMV) infection.

2. Use as claimed in claim 1 in which said aminopeptidase is human aminopeptidase N (hAPN).

3. Use as claimed in claim 1 or claim 2 in which said aminopeptidase is cell free aminopeptidase in water soluble or immobilised form.

4. Use as claimed in any of claims 1 to 3 in which the fragment of an aminopeptidase lacks the site responsible for aminopeptidase activity while retaining HCMV binding activity.

5. Use as claimed in claim 4 in which the aminopeptidase is human aminopeptidase N lacking at least the amino acids at positions 388-392.

6. Use as claimed in claim 4 in which the aminopeptidase is human aminopeptidase N lacking at least the amino acids at positions 360-399.

7. Use as claimed in any of claims 1 to 6 wherein the prevention or treatment of human cytomegalovirus (HCMV) comprises sequentially or concurrently administering to a patient in need thereof
(i) an aminopeptidase or a fragment of an aminopeptidase, and
(ii) an enzyme inhibitor of aminopeptidase.

8. Use as claimed in any of claims 1 to 6 wherein prevention or treatment of human cytomegalovirus (HCMV) comprises sequentially or concurrently administering to a patient in need thereof
(i) an antibody to an aminopeptidase, and
(ii) an enzyme inhibitor of an aminopeptidase said antibody and said enzyme inhibitor being administered in an amount therapeutically effective to provide such prevention or treatment.

9. An in vitro method for the removal of CMV virions or CMV infected hAPN-positive cells from body fluids, blood products or bone marrow by treating said body fluids, blood products or marrow with an antibody to an aminopeptidase or a CMV binding fragment thereof in water-soluble or immobilised form wherein said CMV virions or CMV infected hAPN-positive cells can be partially or completely removed selectively from said body fluids, blood products or bone marrow.

10. An in vitro method for the removal of CMV virions or CMV infected hAPN-positive cells from body fluids, blood products or bone marrow by treating said body fluids, blood products or marrow with cell-free aminopeptidase or fragments thereof in water-soluble or immobilised form wherein said CMV virions or CMV infected hAPN-positive cells can be partially or completely removed selectively from said body fluids, blood products or bone marrow.

11. A method for detecting anti-APN antibodies in a sample from a patient wherein the presence, quantity or quality of anti-APN antibodies in said sample is detected or determined by an immunoassay using an aminopeptidase or fragment thereof as a binding partner for said antibodies.

12. A method as claimed in claim 11, wherein the sample is a body fluid sample.

13. A method as claimed in claim 11 or claim 12 for the diagnosis of CMV infection or the results thereof.

14. A method of diagnosis of CMV infection wherein the presence or quantity of CMV virions is detected or determined by an immunoassay using an antibody against an aminopeptidase or soluble aminopeptidase as a binding partner for said virions.

15. A method of diagnosis of CMV infection wherein cells expressing APN are isolated using an antibody against an aminopeptidase as a binding partner for said APN and the presence or quantity of CMV virions in said cells is subsequently detected or determined.

16. A method as claimed in any of claims 9 to 15 in which said aminopeptidase is human aminopeptidase N.

17. A diagnostic kit for diagnosis of CMV infection by immunoassay to determine the presence or quantity of anti-APN antibodies or of CMV virions in a sample, comprising free or immobilised aminopeptidase or a fragment thereof as a binding partner for anti-APN antibodies and/or a free or immobilised antibody against aminopeptidase or soluble or immobilised APN as binding partner for aminopeptidase on said virions, said kit including labelling means permitting the presence or quantity of said anti-APN antibodies or CMV virions in said sample to be determined.

18. A product containing an aminopeptidase and/or a fragment of an aminopeptidase, and an inhibitor of an aminopeptidase as a combined preparation for simultaneous, separate or sequential use in the prevention or treatment of human cytomegalovirus (HCMV) infection.

19. A product containing an inhibitor of an aminopeptidase and/or an antibody against an aminopeptidase as a combined preparation for simultaneous, separate or sequential use in the prevention or treatment of human cytomegalovirus (HCMV) infection.

## Patentansprüche

1. Verwendung einer Aminopeptidase, eines Fragments einer Aminopeptidase, eines Inhibitors einer Aminopeptidase und/oder eines Antikörpers gegen eine Aminopeptidase zur Herstellung eines Medikaments zur Anwendung bei der Verhütung oder Behandlung einer Human-Cytomegalovirus (HCMV)-Infektion.

2. Verwendung nah Anspruch 1, worin die Aminopeptidase Human-Aminopeptidase N (hAPN) ist.

3. Verwendung nach Anspruch 1 oder Anspruch 2, worin die Aminopeptidase zellfreie Aminopeptidase in wasserlöslicher oder immobilisierter Form ist.

4. Verwendung nah irgendeinem der Ansprüche 1 bis 3, worin dem Fragment einer Aminopeptidase diejenige Stelle fehlt, welche für die Aminopeptidase-Aktivität verantwortlich ist, während die HCMV-bindende Aktivität erhalten geblieben ist.

5. Verwendung nach Anspruch 4, worin die Aminopeptidase Human-Aminopeptidase N ist, der mindestens die Aminosäuren an den Positionen 388-392 fehlen.

6. Verwendung nach Anspruch 4, worin die Aminopeptidase Human-Aminopeptidase N ist, der mindestens die Aminosäuren an den Positionen 360-399 fehlen.

7. Verwendung nach irgendeinem der Ansprüche 1 bis 6, worin die Verhütung oder Behandlung von Human-Cytomegalovirus (HMCV) umfaßt, daß einem Patienten, der dessen bedarf, nacheinander oder gleichzeitig
(i) eine Aminopeptidase oder ein Fragment einer Aminopeptidase und
(ii) ein Enzym-Inhibitor von Aminopeptidase
verabreicht wird,

8. Verwendung nach irgendeinem der Ansprüche 1 bis 6, worin die Verhütung oder Behandlung von Human-Cytomegalovirus (HMCV) umfaßt, daß einem Patienten, der dessen bedarf, nacheinander oder gleichzeitig
(i) ein Antikörper gegen eine Aminopeptidase und
(ii) ein Enzym-Inhibitor einer Aminopeptidase
verabreicht wird, wobei der Antikörper und der Enzym-Inhibitor in einer therapeutisch wirksamen Menge um eine solche Verhütung oder Behandlung zu ergeben, verabreicht werden.

9. Ein in vitro-Verfahren zur Entfernung von CMV-Virionen oder CMV-infizierten, hAPN-positiven Zellen aus Körperflüssigkeiten, Blufprodukten oder Knochenmark durch die Behandlung der Körperflüssigkeiten, Blutprodukte oder des Knochenmarks mit einem Antikörper gegen eine Aminopeptidase oder ein CMV-bindendes Fragment davon in wasserlöslicher oder immobilisierter Form, wobei die CMV-Virionen oder CMV-Infizierten, hAPN-positiven Zellen teilweise oder vollständig aus den Körperflüssigkeiten, Blutprodukten oder dem Knochenmark auf selektive Weise entfernt werden können.

10. Ein in vitro-Verfahren zur Entfernung von CMV-Virionen oder CMV-Infizierten, hAPN-positiven Zellen aus Körperflüssigkeiten, Blutprodukten oder Knochenmark durch die Behandlung der Körperflüssigkeiten, Blutprodukte oder des Knochenmarks mit zellfreier Aminopeptidase oder Fragmenten davon in wasserlöslicher oder immobilisierter Form, wobei die CMV-Virionen oder CMV-infizierten, hAPN-positiven Zellen teilweise oder vollständig aus den Körperflüssigkeiten, Blutprodukten oder dem Knochenmark auf selektive Weise entfernt weiden können.

11. Verfahren zum Nachweis von Anti-APN-Antikörpern in einer Probe von einem Patienten, worin die Anwesenheit, Menge der Qualität von Anti-APN-Antikörpern in der Probe nachgewiesen oder bestimmt wird durch einen Immunoassay unter Verwendung einer Aminopeptidase oder eines Fragments davon als Bindungspartner für die Antikörper.

12. Verfahren nach Anspruch 11, worin die Probe eine Körperflüssigkeitsprobe ist.

13. Verfahren nach Anspruch 11 oder Anspruch 12 zur Diagnose von CMV-Infektion oder deren Folgen.

14. Verfahren zur Diagnose von CMV-Infektion, worin die Anwesenheit oder Menge von CMV-Virionen nachgewiesen oder bestimmt wird durch einen Immunoassay unter Verwendung eines Antikörpers gegen eine Aminopeptidase oder von löslicher Aminopeptidase als Bindungspartner für die Virionen.

15. Verfahren zur Diagnose von CMV-Infektion, worin APN-exprimierende Zellen unter Verwendung eines Antikörpers gegen eine Aminopeptidase als Bindungspartner für die APN isoliert werden und anschließend die Anwesenheit oder Menge von CMV-Virionen in den Zellen nachgewiesen oder bestimmt wird.

16. Verfahren nach irgendeinem der Ansprüche 9 bis 15, worin die Aminopeptidase Human-Aminopeptidase N ist.

17. Diagnostischer Kit für die Diagnose von CMV-Infektion mittels Immunoassay zur Bestimmung der Anwesenheit oder Menge von Anti-APN-Antikörpern oder CMV-Virionen in einer Probe, umfassend freie oder immobilisierte Aminopeptidase oder ein Fragment davon als Bindungspartener für Anti-APN-Antikörper und/oder einen freien oder immobilisierten Antikörper gegen Aminopeptidase oder lösliche oder immobilisierte APN als Bindungspartner für Aminopeptidase auf den Virionen, wobei der Kit Markierungsmittel umfaßt, welche die Bestimmung der Anwesenheit oder Menge der Anti-APN-Antikörper oder CMV-Vironen in der Probe erlauben.

18. Produkt, enthaltend eine Aminopeptidase und/oder ein Fragment einer Aminopeptidase und einen Inhibitor einer Aminopeptidase als kombiniertes Präparat für die gleichzeitige, separate oder nacheinander erfolgende Anwendung bei der Verhütung oder Behandlung einer Human-Cytomegalovirus (HCMV)-Infektion.

19. Produkt, enthaltend einen Inhibitor einer Aminopeptidase und/oder einen Antikörper gegen eine Aminopeptidase als kombiniertes Präparat für die gleichzeitige, separate oder nacheinander erfolgende Anwendung bei der Verhütung oder Behandlung einer Human-Cytomegalovirus (HMCV)-Infektion.

## Revendications

1. Utilisation d'une aminopeptidase, d'un fragment d'une aminopeptidase, d'un inhibiteur d'une aminopeptidase et/ou d'un anticorps dirigé contre une aminopeptidase dans la fabrication d'un médicament destiné à la prévention ou au traitement de l'infection par le cytomégalovirus humain (HCMV).

2. Utilisation selon la revendication 1, ladite aminopeptidase étant l'aminopeptidase N humaine (hAPN).

3. Utilisation selon la revendication 1 ou selon la revendication 2, ladite aminopeptidase étant une aminopeptidase acellulaire sous forme hydrosoluble ou immobilisée.

4. Utilisation selon l'une des revendications 1 à 3, ledit fragment d'une aminopeptidase ne possédant pas le site responsable de l'activite aminopeptidasique tout en conservant l'activité de liaison à l'HCMV.

5. Utilisation selon la revendication 4, ladite aminopeptidase étant une aminopeptidase N humaine à laquelle il manque au moins les acides aminés occupant les positions 388 à 392.

6. Utilisation selon la revendication 4, ladite aminopeptidase étant une aminopeptidase N humaine à laquelle il manque au moins les acides aminés occupant les positions 360 à 399.

7. Utilisation selon l'une des revendications 1 à 6, dans laquelle la prévention ou le traitement du cytomégalovirus humain (HCMV) comprend l'administration séquentielle ou simultanée à un patient qui en a besoin de
(i) une aminopeptidase ou un fragment d'une aminopeptidase, et
(ii) un inhibiteur enzymatique d'une aminopeptidase.

8. Utilisation selon l'une des revendications 1 à 6, dans laquelle la prévention ou le traitement du cytomégalovirus humain (HCMV) comprenant l'administration séquentielle ou simultanée à un patient qui en a besoin de
(i) un anticorps dirigé contre une aminopeptidase, et
(ii) un inhibiteur enzymatique d'une aminopeptidase, ledit anticorps et ledit inhibiteur enzymatique etant administrés en quantité thérapeutiquement efficace pour pourvoir à cette prévention ou a ce traitement.

9. Procédé d'élimination in vitro de virions de CMV ou de cellules hAPN-positives infectées par le CMV dans des liquides corporels, des produits sanguins ou de la moelle osseuse par un traitement desdits liquides corporels produits sanguins ou moelle osseuse par un anticorps dirigé contre une aminopeptidase ou un fragment d'aminopeptidase qui se lie au CMV sous une forme hydrosoluble ou immobilisée, lesdits virions de CMV ou cellules hAPN-positives infectées par le CMV pouvant être partiellement ou complètement éliminés, de manière sélective, dans lesdits liquides corporels, produits sanguins ou moelle osseuse.

10. Procédé d'élimination in vitro de virions de CMV ou de cellules hAPN-positives infectées par le CMV dans des liquides corporels des produits sanguins ou de la moelle osseuse par un traitement desdits liquides corporels, produits sanguins ou moelle osseuse par une aminopeptidase acellulaire ou des fragments de celle-ci sous forme hydrosoluble ou immobilisée, lesdits virions de CMV au cellules hAPN-positives infectées par le CMV pouvant être partiellement ou complètement éliminés sélectivement, de manière sélective, dans lesdits liquides corporels, produits sanguins ou moelle osseuse.

11. Procédé de détection d'anticorps anti-APN dans un échantillon prélevé sur un patient, dans lequel la présence, la quantité ou la qualité d' anticorps anti-APN dans ledit échantillon est détectée ou déterminée à l'aide d'un dosage immunologique utilisant une aminopeptidase ou un fragment d'aminopeptidase en tant que partenaire de liaison pour lesdits anticorps.

12. Procédé selon la revendication 11, l'échantillon étant un échantillon d'un liquide corporel.

13. Procédé selon la revendication 11 ou la revendication 12 pour le diagnostic de l'infection par le CMV ou des résultats de celle-ci.

14. Procédé de diagnostic de l'infection par le CMV dans lequel la présence ou la quantité de virions de CMV est détectée ou dosée à l'aide d'un essai immunologique en utilisant un anticorps dirigé contre une aminopeptidase ou une aminopeptidase soluble en tant que partenaire de liaison pour lesdits virions.

15. Procédé de diagnostic de l'infection par le CMV dans lequel les cellules exprimant l'APN sont isolées à l'aide d'un anticorps dirigé contre une aminopeptidase en tant que partenaire de liaison pour ladite APN et la présence ou la quantité de virions de CMV dans lesdites cellules est ensuite détectée ou dosée.

16. Procédé selon l'une des revendications 9 à 15 dans lequel ladite aminopeptidase est l'aminopeptidase N humaines.

17. Kit diagnostique pour le diagnostic de l'infection par le CMV au moyen d'un test immunologique permettant de déterminer la présence ou la quantité d'anticorps anti-APN ou de virions de CMV dans un échantillon, ou de les doser, comprenant une aminopeptidase ou un fragment d'aminopeptidase libre ou immobilisé en tant que partenaire de liaison pour les anticorps anti-APN et/ou un anticorps libre ou immobilisé dirigé contre une aminopeptidase ou une APN soluble ou immobilisée en tant que partenaire de liaison pour l'aminopeptidase sur lesdits virions, ledit kit incluant un moyen de marquage permettant de déterminer la présence ou la quantité desdits anticorps anti-APN ou des virions de CMV ou de les doser dans ledit échantillon.

18. Produit contenant une aminopeptidase et/ou un fragment d'aminopeptidase et un inhibiteur d'une aminopeptidase sous la forme d'une préparation combinée destinée à être utilisée de manière simultanée, indépendante ou séquentielle dans la prévention ou le traitement de l'infection par le cytomégalovirus humain (HCMV).

19. Produit contenant un inhibiteur d'une aminopeptidase et/ou un anticorps dirigé contre une aminopeptidase sous la forme d'une préparation combinée destinée à être utilisée de manière simultanée, indépendante ou séquentielle dans la prévention ou le traitement de l'infection par le cytomégalovirus humain (HCMV).
